# EUROPEAN PATENT SPECIFICATION

(11) **EP 1 581 537 B1**
(45) Date of publication and mention of the grant of the patent: **21.11.2007**
(21) Application number: 03768031.1
(22) Date of filing: 15.12.2003
(51) Int. Cl.: C07D 495/04

(54) **Process for removing impurity S from olanzapine polymorphic form I**
Prozess zum Entfernen von Verunreinigung S von Olanzapin polymorpher Form I
Procédé pour éliminer d'impureté S d'une forme polymorph I d'olanzapine

(30) Priority: 20.12.2002 PL 35792802
(43) Date of publication of application: 05.10.2005
(73) Proprietor: Adamed SP. Z O.O., 05-152 Czosnów k/Warszawy (PL)
(72) Inventor: MAJKA, Zbigniew, 39-102 Lubzina (PL); STAWINSKI, Tomasz, 05-462 Wiazowna (PL)
(74) Representative: Sitkowska, Jadwiga
(86) International application number: PCT/IB2003/005931
(87) International publication number: WO 2004/056833

(56) References cited:
- EP-A- 0 733 367
- WO-A-02/18390
- WO-A-03/097650
- WO-A-03/101997
- WO-A-20/04006933
- US-A- 5 229 382
- US-A- 5 703 232
- US-A- 5 736 541
- US-B1- 6 348 458
- FURNISS, B. S. ET AL.: "Vogels's Textbook of Practical Organic Chemistry" 1996, JOHN WILEY & SONS, INC. , NEW YORK Pages 135-140.
- Experimental Report of The Warsaw University of Technology, Faculty of Chemistry to Adamed sp.z o.o., dated 27.09.2002, in Polish language (6 pages); & English translation thereof (2 pages).
- Report of Dr. Z. Kaszkur (Institute of Physical Chemistry of the Polish Academy of Sciences) to Adamed sp.z o.o., dated 08.06.2005, in Polish language (1 page); & English translation thereof (1 page).

## Description

The present invention relates to a process removing impurity S (1-(chloromethyl)-1-methyl-4-(2-methyl-10H-thieno[2,3-b][1,5]benzodiazepin-4-yl)piperazin-1-ium chloride) from olanzapine form I.

Olanzapine (2-methyl-4-[4-methyl-1-piperazinyl]-10H-thieno[2.3-b]-[1.5]benzodiazepine) is a known medicine acting on the central nervous system. The "polymorphic form I" herein represents the form I as defined in WO96/30375. The X-ray diffractogram of this polymorphic form exhibits the characteristic interplanar distances d (in angstrems): 9.94, 8.55, 8.24, 6.88, 6.37, 6.24, 5.58, 5.30, 4.98, 4.83, 4.72, 4.62, 4.53, 4.46, 4.29, 4.23, 4.08, 3.82, 3.74, 3.69, 3.58, 3.50, 3.33, 3.28, 3.21, 3.11, 3.05, 2.94, 2.81, 2.75, 2.65, 2.63, 2.59.

The patent application EP0733367 discloses that olanzapine polymorphic form II is converted into another polymorphic form when contacted with methylene chloride.

International patent application No. WO02/18390 discloses a process for the preparation of the polymorphic form I of olanzapine by crystallisation of raw olanzapine, olanzapine hydrate or its polymorphic form II from methylene chloride. The process as described and claimed therein comprises dissolving olanzapine or the hydrate thereof or the form II, in methylene chloride at reflux temperature, the treatment of the hot solution with carbon, filtering off the solution while hot, crystallizing of the form I by cooling the solution and filtering off the form I.

It has been found that extending the duration of the process of olanzapine crystallization from methylene chloride to yield sufficiently pure final crystalline form I, leads to formation of additional contaminating compound, especially during heating at reflux temperature. Said compound (1-(chloromethyl)-1-methyl-4-(2-methyl-10H-thieno[2.3-b]-[1.5]benzodiazepin-4-yl)piperazin-1-ium chloride) referred sometimes also as the "impurity S" is a product of quaternization of a basic nitrogen atom of piperazine ring with methylene chloride. It has been also found that it can not be removed efficiently by repeated crystallization from methylene chloride. Repeated crystallizations from methylene chloride lead only to a progressive accumulation of the impurity to levels unacceptable in view of the purity requirements for the pharmaceutical materials. According to the requirements of International Conference of Harmonisation (ICH) the impurity level must not exceed 0,15%. Otherwise, toxicological characterization of the contaminating molecule is required. The removal of the impurity S on a large scale constitutes therefore a serious technical problem.

The process for the olanzapine crystallization from methylene chloride, as disclosed in WO 02/18390, comprises treating a hot solution of olanzapine in methylene chloride with carbon, before carrying out the crystallization.

However, the present inventors have found that treating the hot solution of olanzapine with carbon provides neither the removal of the said undesirable impurity nor even lowering its level.

Surprisingly, it has been found that the impurity S can be efficiently removed, if the solution of olanzapine is subjected to a treatment with silica gel, by contacting said solution with silica gel, before performing crystallization.

Thus, the invention provides a process for removing impurity S (1-(chloromethyl)-1-methyl-4-(2-methyl-10H-thieno[2,3-b][1,5]benzodiazepin-4-yl)piperazin-1-ium chloride) from olanzapine polymorphic form I, wherein said form I is as defined in WO96/30375, comprising treatment of solution of olanzapine in methylene chloride with silica gel and then crystallization.

Preferably, in a first and preferred variant of the above process, a hot solution of olanzapine is contacted with silica gel, especially at reflux temperature.

This may be performed by adding silica gel to the hot olanzapine solution in methylene chloride, and heating at reflux temperature to dissolve the solid, followed by adding silica gel to the hot solution of olanzapine in methylene chloride and heating at reflux temperature with vigorous stirring. The heating lasts usually several minutes. After the treatment is complete, the silica gel is filtered off while hot and the solution is cooled to enable the crystallization of olanzapine, followed by filtering off the crystallized olanzapine.

Advantageously, the above variant comprises:
a) combining olanzapine with methylene chloride;
b) heating olanzapine with methylene chloride at reflux temperature to form a solution;
c) adding silica gel to the hot solution of olanzapine in methylene chloride and stirring at reflux temperature;
d) filtering off the silica gel from said solution while hot; and
e) cooling the filtrate and collecting the crystallized form I of olanzapine.

According to the second embodiment of this first variant, silica gel is added to the solution of olanzapine in methylene chloride at room temperature and then the solution is heated at reflux temperature, preferably with vigorous stirring. The heating lasts usually several minutes. Since the cold dissolution requires greater amounts of methylene chloride, the crystallization will require the partial evaporation of methylene chloride carried out under the reduced pressure to limit the forming of the impurity S once again.

Advantageously, the above variant comprises:
a) dissolving olanzapine in methylene chloride at room temperature;
b) adding silica gel to the solution of olanzapine in methylene chloride at room temperature;
c) heating and stirring the solution of olanzapine in methylene chloride with silica gel at reflux temperature;
d) filtering off the silica gel while hot; and
e) reducing the volume of the filtrate under reduced pressure, cooling the filtrate and filtering off the crystallized form I of olanzapine.

The inventors have found that according to the process of the invention, when the treatment is performed at reflux, the contents of the impurity S can be reduced more than 10 times.

According to the third variant of the above process, contacting the solution of olanzapine with silica gel may be carried out without heating the solution, with the use of a chromatographic column packed with silica gel and passing the solution of olanzapine through the column at the room temperature, for example in the flash chromatography conditions. In this case the greater amounts of methylene chloride are also required to dissolve olanzapine at the room temperature, and therefore a concentration of olanzapine solution will be needed before crystallization, as described hereinbefore.

In the process according to the invention, as a starting olanzapine a purified olanzapine may be used. However, the results are also good in the case of a raw (technical) olanzapine, such as e.g. olanzapine prepared in the reaction of 2-(2-nitroanilino)-5-methyltiophene-3-carbonitrile with stannous chloride in an aqueous-alcoholic solution in the presence of the hydrochloric acid, followed by the reaction of 4-amino-2-methyl-10H-thieno[2,3-b][1,5]benzodiazepine thus formed with N-methylpiperazine, as disclosed in the Polish Patent Application No P-350717.

Generally, the amount of the silica gel used is 1 do 10 percent by weight with respect to olanzapine to be purified.

By the process of the invention there is provided a pharmaceutically pure polymorphic form I of olanzapine, comprising below 0.15% by weight of impurity S (1-(chloromethyl)-1-methyl-4-(2-methyl-10H-thieno[2.3-b][[1.5]benzodiazepin-4-yl)piperazin-1-ium chloride), advantageously about 0.05% by weight of impurity S.

In an embodiment of the invention a process of removing of S impurity from olanzapine polymorphic form I comprises treatment of solution of olanzapine form I in methylene chloride with silica gel and then crystallization.

The invention will be now further described in the following, nonlimiting examples.

### Example 1.

### Preparation of the polymorphic form I from the raw olanzapine

400 g of the raw olanzapine (99% HPLC) was heat dissolved in 3000 ml of methylene chloride. After dissolving, 20 g of a silica gel (230-400 Mesh) was added. The mixture was heat stirred for 5 min. and filtered. After cooling to about 0°C the separated crystalline olanzapine was filtered off. Purity: 99.92% (HPLC).

### Example 2

### Purification of olanzapine after the crystallization from methylene chloride

300 g of olanzapine previously crystallized from methylene chloride (form I), containing 0.6% of 1-(chloromethyl)-1-methyl-4-(2-methyl-10H-thieno[2.3-b][1.5]benzodiazepin-4-yl)piperazin-1-ium chloride was dissolved in 2100 ml of methylene chloride while heating. 30 g of silica gel (230-400 Mesh) was added. The mixture was stirred with boiling for 5 min. and filtered while hot. The filtrate was cooled to about 0°C, and the crystallized olanzapine was filtered off and dried. Impurity S content: 0.05% (HPLC).

## Claims

1. A process for removing impurity S (1-(chloromethyl)-1-methyl-4-(2-methyl-10H-thieno[2,3-b][1,5]benzodiazepin-4-yl)piperazin-1-ium chloride) from olanzapine polymorphic form I, wherein said form I is as defined in WO96/30375, comprising treatment of solution of olanzapine in methylene chloride with silica gel and then crystallization.

2. The process according to claim 1, **characterized in that** it comprises hot treatment with silica gel at reflux temperature.

3. The process according to claim 2, **characterized in that** it comprises:
a) combining olanzapine with methylene chloride;
b) heating olanzapine with methylene chloride at reflux temperature to form a hot solution;
c) adding silica gel to the hot solution of olanzapine in methylene chloride and then stirring at reflux temperature;
d) filtering off the silica gel from said solution while hot; and
e) cooling the filtrate and collecting crystallized form I of olanzapine.

4. The process according to claim 2, **characterized in that** the silica gel is added to the solution of olanzapine form I in methylene chloride at room temperature and then the solution is heated at reflux.

5. The process according to claim 2, **characterized in that** it comprises:
a) dissolving olanzapine in methylene chloride at room temperature;
b) adding silica gel to the solution of olanzapine in methylene chloride at room temperature;
c) heating and stirring the solution of olanzapine in methylene chloride with silica gel at reflux temperature;
d) filtering off the silica gel from said solution while hot; and
e) cooling the filtrate and collecting the crystallized form I of olanzapine.

6. The process according to claim 1, **characterized in that** said treatment of the solution of olanzapine with silica gel is carried out at room temperature.

7. The process according to claim 6, **characterized in that** it comprises passing the solution of olanzapine in methylene chloride through a column packed with silica gel, to give a filtrate.

8. The process according to claim 6, **characterized in that** it comprises adding silica gel to the solution of olanzapine in methylene chloride at room temperature and stirring the mixture thus obtained at room temperature, followed by filtering the gel off at room temperature, to give a filtrate.

9. The process according to claims 7 or 8, **characterized in that** the filtrate is concentrated under reduced pressure, cooled and the crystallized form I of olanzapine is filtered off.

10. The process according to any one of claims 1 to 9, **characterized in that** the amount of the silica gel is 1 to 10 percent by weight with respect to olanzapine being purified.

11. The process according to any one of claims 1 to 10, **characterized in that** said solution of olanzapine in methylene chloride is a solution of olanzapine form I.

## Patentansprüche

1. Verfahren zur Entfernung von S Verunreinigung (1-(chloromethyl)-1-methyl-4-(2-methyl-10H-thieno[2,3-b][1,5]benzodiazepin-4-yl)piperazin-1-ium Chlorid) aus polymorpher Form I von Olanzapin, worin besagte Form I wie in WO96/30375 definiert ist, umfassend Behandlung einer Lösung von Olanzapin im Methylenchlorid mit Silikagel und dann Kristallisation.

2. Verfahren nach Anspruch 1, **dadurch gekennzeichnet, dass** es heiße Behandlung mit Silikagel bei Rückflusstemperatur umfasst.

3. Verfahren nach Anspruch 2, **dadurch gekennzeichnet, dass** es umfasst:
a) Kombinieren von Olanzapin mit Methylenchlorid;
b) Erhitzen von Olanzapin mit Methylenchlorid bei Rückflußtemperatur, um eine heiße Lösung zu erhalten;
c) Zugabe des Silikagels zu der heißen Lösung von Olanzapin im Methylenchlorid und anschließend Rühren bei Rückflußtemperatur;
d) Herausfiltrieren des Silikagels aus besagter Lösung, solange heiße; und
e) Kühlen des Filtrats und Einsammeln die auskristallisierende Form I von Olanzapin.

4. Verfahren nach Anspruch 2, **dadurch gekennzeichnet, dass** das Silikagel zu der Lösung von Olanzapin im Methylenchlorid bei Raumtemperatur zugegeben wird und dann die Lösung bei Rückflußtemperatur erhitzt wird.

5. Verfahren nach Anspruch 2, **dadurch gekennzeichnet, dass** es umfasst:
a) Auflösen von Olanzapin im Methylenchlorid bei Raumtemperatur;
b) Zugabe des Silikagels zu der Lösung von Olanzapin im Methylenchlorid bei Raumtemperatur;
c) Erhitzen und Rühren der Lösung von Olanzapin im Methylenchlorid mit Silikagel bei Rückflusstemperatur;
d) Herausfiltrieren des Silikagels aus besagter Lösung, solange heiße; und
e) Kühlen des Filtrats und Einsammeln die auskristallisierende Form I von Olanzapin.

6. Verfahren nach Anspruch 1, **dadurch gekennzeichnet dass** besagte Behandlung der Lösung von Olanzapin mit Silikagel bei Raumtemperatur durchgeführt wird.

7. Verfahren nach Anspruch 6, **dadurch gekennzeichnet, dass** es das Passieren der Lösung von Olanzapin im Methylenchlorid durch eine mit Silikagel gepackte Säule umfasst, um ein Filtrat zu erhalten.

8. Verfahren nach Anspruch 6, **dadurch gekennzeichnet, dass** es Zugabe des Silikagels zu der Lösung von Olanzapin im Methylenchlorid bei Raumtemperatur und Rühren der so erhaltenen Mischung bei Raumtemperatur, anschließend Herausfiltrieren des Silikagels bei Raumtemperatur umfasst, um ein Filtrat zu erhalten.

9. Verfahren nach Anspruch 7 oder 8, **dadurch gekennzeichnet, dass** das Filtrat unter vermindertem Druck konzentriert, gekühlt und die auskristallisierende Form I von Olanzapin herausfiltriert werden.

10. Verfahren nach einem der Ansprüche 1 bis 9, **dadurch gekennzeichnet, dass** die Menge des Silikagels bezogen auf den zu reinigenden Olanzapin von 1 bis 10 Gew.-% beträgt.

11. Verfahren nach einem der Ansprüche 1 bis 9, **dadurch gekennzeichnet, dass** gesagte Lösung von Olanzapin im Methylenchlorid die Lösung von Form I von Olanzapin ist.

## Revendications

1. Procédé pour éliminer l'impureté S (le chlorure de (1-(chlorométhyl)-1-méthyl-4-(2-méthyl-10H-thiéno[2.3-b][1.5]benzodiazépin-4-yl)-pipérazin-1-ium) d'une forme polymorphique I d'olanzapine, ladite forme polymorphique I etant définie comme dans WO96/30375, qui comprend le traitement d'une solution d'olanzapine dans le chlorure de méthyléne avec du silicagel et puis la cristallisation.

2. Procédé selon la revendication 1 **caractérisé en ce qu'**il comprend le traitement à chaud avec du silicagel à la temperature de reflux.

3. Procédé selon la revendication 2 **caractérisé en ce qu'**il comprend:
a) le mélangeage d'olanzapine avec du chlorure de méthyléne;
b) le chauffage d'olanzapine avec du chlorure de méthyléne à la temperature de reflux pour obtenir une solution chaude;
c) l'addition du silicagel à la solution chaude d'olanzapine dans le chlorure de méthyléne et puis l'agitation à la temperature de reflux;
d) la filtration du silicagel de la solution etant encore chaude; et
e) le réfroidisement du filtrat et la récupération la forme I crystallisée.

4. Procédé selon la revendication 2 **caractérisé en ce que** le silicagel est ajouté à la solution de la forme polymorphique I d'olanzapine dans le chlorure de méthyléne à la témperature ambiante et puis la solution est chauffée à la temperature de reflux.

5. Procédé selon la revendication 2 **caractérisé en ce qu'**il comprend:
a) la dissolution d'olanzapine dans le chlorure de méthyléne à la témperature ambiante;
b) l'addition du silicagel à la solution d'olanzapine dans le chlorure de méthyléne à la témperature ambiante;
c) le chauffage et l'agitation de la solution d'olanzapine dans le chlorure de méthyléne à la temperature de reflux ;
d) la filtration du silicagel de la solution etant encore chaude; et
e) le réfroidisement le filtrat et la récupération de la forme I crystallizée.

6. Procédé selon la revendication 1 **caractérisé en ce que** ledit traitement de la solution d'olanzapine avec du silicagel est realisé à la témperature ambiante.

7. Procédé selon la revendication 6 **caractérisé en ce qu'**il comprend le passage de la solution d'olanzapine dans le chlorure de méthyléne au travers d'une colonne rempliée du silicagel pour obtenir le filtrat.

8. Procédé selon la revendication 6 **caractérisé en ce qu'**il comprend l'addition du silicagel à la solution d'olanzapine dans le chlorure de méthyléne à la témperature ambiante et l'agitation d'une mélange ainsi obtenue à la témperature ambiante, suivie par filtration du silicagel à la témperature ambiante pour obtenir le filtrat.

9. Procédé selon la revendication 7 ou 8 **caractérisé en ce que** le filtrat est concentré sous la pression reduite, réfroidi et la forme I d'olanzapine cristallisée est filtrée.

10. Procédé selon l'une quelconque des revendications 1 à 9, **caractérisé en ce que** la quantité du silicagel est de 1 à 10 pour cent en poids par rapport à d'olanzapine etant purifiée.

11. Procédé selon l'une quelconque des revendications 1 à 9, **caractérisé en ce que** ladite solution d'olanzapine dans le chlorure de méthyléne est une solution de la forme I d'olanzapine.
